# EUROPEAN PATENT APPLICATION

(11) **EP 4 144 287 A1**
(43) Date of publication of application: **08.03.2023**
(21) Application number: 21194238.8
(22) Date of filing: 01.09.2021
(51) Int. Cl.: A61B 5/00

(54) **BLUETOOTH LOW ENERGY PRESSURE SENSOR**

(71) Applicant: BIOTRONIK SE & Co. KG, 12359 Berlin (DE)
(72) Inventor: DÖRR, Thomas, 12437 Berlin (DE); MUESSIG, Dirk, West Linn, 97068 (US)
(74) Representative: Biotronik Corporate Services SE

(57) **Abstract**

The present disclosure relates to intra-body pressure determination. For example, a method for intra-body pressure determination is provided. The method comprises acquiring first pressure data relating to the intra-body pressure by an implantable pressure sensor. Further, it comprises wirelessly transmitting the first pressure data to an implantable device. Finally, it also comprises wirelessly relaying, by the implantable device, the first pressure data to a mobile communication apparatus for correction of the first pressure data.

## Description

The present disclosure generally relates to determining an intra-body pressure, e.g. a blood pressure, and corresponding systems, methods, devices, and computer programs.

Implantable pressure sensors have been known for years. They can be implanted, e.g. into a heart and/or a blood vessel to monitor cardiac and/or vascular blood pressure, respectively, at various locations, for example. Also for numerous other applications implantable pressure sensors exist, such as for measuring pressure in the spine, joints, the bladder, etc.

All implantable pressure sensors face challenges with at least two aspects, namely how to provide the data acquired by the (intra-body) sensor to the outside world (e.g. the patient, doctor, etc.), and how to calibrate the acquired data to obtain reliable intra-body pressure values.

Over the years, various approaches have been developed to address the above two aspects. However, despite substantial efforts, the known approaches to address these aspects are still less than optimal. Since intra-body pressure sensing is of paramount importance for monitoring patient health and quickly reacting in emergency situations, further improvements may improve quality of life for patients and also prevent life-threatening situations. There is therefore a need to improve the known ways to determine an intra-body pressure.

The above need is at least in part met by the aspects described herein.

According to a first aspect, a method is provided for determining an intra-body pressure. The method comprises the following steps: acquiring first pressure data relating to the intra-body pressure by an implantable pressure sensor; wirelessly transmitting the first pressure data to an implantable device; and wirelessly relaying, by the implantable device, the first pressure data to a mobile communication apparatus for correction of the first pressure data.

The above method in particular allows using a general purpose mobile communication apparatus, such as a general purpose tablet or smartphone, to receive and correct the first pressure data from the implantable pressure sensor. This is enabled by using an implantable device as a wireless relay between the implantable sensor and the mobile communication apparatus. Thus, the distance which wireless signals have to traverse through the patient's body to wirelessly reach the mobile communication apparatus from the wireless relay is greatly reduced, as the implantable device may be implanted close to a surface of the body, whereas the implantable pressure sensor is typically implanted more deeply within the body. Hence, general purpose communication protocols such as Bluetooth (e.g. Bluetooth Low Energy), Near Field Communication (NFC), etc. may be used that are generally supported by general purpose mobile communication apparatuses, although they typically suffer strong losses when transmission occurs through a patient's body. As a result, dedicated external devices that, e.g., operate with less common protocols, such as the Medical Implant Communication Service (MICS) that provides a spectral range that allows communications with low absorption by the patient's body and thus over a longer distance, may not be needed. This may strongly reduce costs and increase ease of use by the patient associated with implantable pressure sensors (that may be implemented as sensors for sensing a physiological pressure, e.g. a blood pressure, for example).

At the same time, this solution allows conveniently correcting the first pressure data by the mobile communication apparatus. For example, even general purpose mobile communication apparatuses typically include a pressure sensor which allows measuring the ambient (barometric) pressure around the patient based on which the first pressure data may be corrected. For example, the first pressure data may be corrected based on an ambient pressure, e.g. the difference between a pressure indicated by the first pressure data and the ambient pressure as measured by the mobile communication apparatus may be considered in this regard.

Hence, in combination, the above aspect allows not only doing away with an additional dedicated patient device (a general purpose mobile communication apparatus is carried by virtually any patient anyway), but at the same time allows determining intra-body pressure with the same level of accuracy as with dedicated devices, by making use of a simple functionality (generally provided by general purpose mobile communication apparatuses).

In an example, the method may further comprise acquiring, by the mobile communication apparatus, second pressure data by a pressure sensor of the mobile communication apparatus. Moreover, it may include correcting the first pressure data at least in part based on the second pressure data and/or forwarding the first and second pressure data for correcting to a remote communication system. In short, the first pressure data may be corrected for variations due to, e.g., ambient pressure variations that are reflected in second pressure data by a corresponding ambient pressure sensor of the mobile communication apparatus. The correction may be carried out, e.g. directly by the mobile communication apparatus. Additionally or alternatively, the correction may be carried out by a remote communication system based on the first and second data forwarded by the mobile communication apparatus.

A second aspect of the present disclosure relates to a system for determining an intra-body pressure. The system may comprise an implantable pressure sensor for acquiring first pressure data relating to the intra-body pressure. Moreover, the system may comprise an implantable device comprising means for wirelessly receiving the first pressure data from the implantable pressure sensor and for wirelessly relaying the first pressure data to a mobile communication apparatus for correction of the first pressure data. This system may facilitate determining intra-body pressure in an accurate, but at the same time cost-reduced manner and more conveniently, as outlined above with respect to the first aspect.

The system may further comprise the mobile communication apparatus. The mobile communication apparatus may comprise a pressure sensor for acquiring second pressure data. The mobile communication apparatus may further comprise means for correcting the first pressure data at least in part based on the second pressure data and/or for forwarding the first and second pressure data for correcting to a remote communication system.

A third aspect of the present disclosure relates to a method for determining an intra-body pressure, carried out by a mobile communication apparatus. The method may comprise the following steps: wirelessly receiving, from a relay device, first pressure data relating to the intra-body pressure acquired by an implantable pressure sensor; receiving second pressure data acquired by a pressure sensor of the mobile communication apparatus; correcting the first pressure data at least in part based on the second pressure data and/or forwarding the first and second pressure data for correcting to a remote communication system.

The above feature combination harnesses a simple functionality typically provided by a general purpose mobile communication apparatus. It may thus render a dedicated patient device superfluous but at the same time ensure accurate determination of the intra-body pressure.

The wirelessly receiving may comprise wirelessly receiving via at least one of: Bluetooth (for example Bluetooth Low Energy) and Near Field Communication (NFC; such as defined by ISO 18092 or ECMA 340). The concept of receiving the first pressure data not directly from the implantable pressure sensor (that may be deeply implanted and thus may not be suitable for Bluetooth and/or NFC), but instead from a relay device, allows using communication protocols such as Bluetooth and/or NFC that are typically available in mobile communication apparatuses such that no specific additional hardware is needed to implement the above aspect.

In an example, the mobile communication apparatus may be a handheld apparatus, such as a phone, a tablet, etc., in particular a smartphone. The conceptually novel approach as described herein particularly allows realizing the described aspects with a general purpose handheld apparatus, such as a general purpose smartphone. The corresponding functionality may simply be provided by an app, downloadable from an app store. Instead of as a handheld apparatus, the mobile communication apparatus may also be implemented as a smartwatch, for example.

It may particularly be beneficial that the mobile communication apparatus may thus be carried by the patient at all times. Typically, it is also always on (particularly in case of a smartphone or smartwatch), such that it may be adapted to acquire the second pressure data continuously. It may thus be possible to correct any first pressure data with corresponding second pressure data (e.g. relating to the same time), even if the first pressure data is only received after it has been acquired by the implantable pressure sensor (for example, the pressure sensor may acquire first pressure data for various periods of time, but the first pressure data may only be received by the mobile communication apparatus at a later point in time).

In an example, the relay device may be an implantable device. For example, the relay device may be adapted to be implanted close to the surface of the patient's body, e.g. subcutaneously, etc. This allows using a relay device that provides a reliable gateway for the implantable pressure sensor to the outside world with low communication losses.

It is generally noted that whenever the term implantable is used in the present disclosure, e.g. referring to an implantable pressure sensor or an implantable device, also the notion of the respective unit being implanted is understood to be comprised. In other words, an implantable pressure sensor or an implantable device may relate to an implanted pressure sensor or an implanted device. Alternatively, the pressure sensor or device may not yet be implanted.

A fourth aspect relates to a computer program. The computer program may comprise instructions that, when carried out by a processor of a mobile communication apparatus, cause the mobile communication apparatus to implement the following steps: wirelessly receiving, from a relay device, first pressure data relating to the intra-body pressure acquired by an implantable pressure sensor; receiving second pressure data acquired by a pressure sensor of the mobile communication apparatus; correcting the first pressure data at least in part based on the second pressure data and/or forwarding the first and second pressure data for correcting to a remote communication system. The computer program may be stored on a computer-readable medium, e.g. on a computer readable medium of the mobile communication apparatus, for example. It may be provided, e.g., as a (data evaluation) app, e.g. downloadable from an app store.

In an example, a mobile communication apparatus for determining an intra-body pressure is provided that comprises means for wirelessly receiving, from a relay device, first pressure data relating to the intra-body pressure acquired by an implantable pressure sensor. It may further comprise means for receiving second pressure data acquired by a pressure sensor of the mobile communication apparatus. The mobile communication apparatus may further comprise means for correcting the first pressure data at least in part based on the second pressure data and/or means for forwarding the first and second pressure data for correcting to a remote communication system.

A fifth aspect relates to a method for assisting in determining an intra-body pressure, carried out by an implantable device. The method may comprise: wirelessly receiving, from an implantable pressure sensor, first pressure data relating to the intra-body pressure acquired by the implantable pressure sensor; wirelessly relaying the first pressure data to a mobile communication apparatus for correction of the first pressure data. This method may allow reliably correcting pressure data with a general purpose mobile communication apparatus, such that accurate intra-body sensing may be provided with reduced costs, as outlined herein.

In an example, the methods described herein may be implemented such that the wirelessly relaying comprises wirelessly relaying via at least one of: Bluetooth (e.g., Bluetooth Low Energy) and Near Field Communication (NFC). Using these protocols, that may be implemented by general purpose mobile communication apparatuses, may be enabled by the implantable device that relays the first data of the implantable pressure sensor (that may be deeply implanted and thus may not be suitable for Bluetooth and/or NFC) to the (external) mobile communication system, as outlined herein.

In an example, the methods outlined herein may be implemented such that the mobile communication apparatus is embodied as a handheld apparatus (e.g. a tablet, phone, etc.), in particular a smartphone and/or as a smartwatch. This is enabled by the combination of features as outlined above. Using a handheld device and/or a smartwatch may be convenient for the patient and, particularly using general purpose devices (e.g. a tablet or smartphone), may reduce the number of devices the patient has to carry, pay for, and/or regularly update for security reasons.

In a further example, the implantable pressure sensor comprises a blood pressure sensor, in particular an intravascular blood pressure sensor. Hence, blood pressure may be determined accurately in a convenient and cost-effective manner. The pressure sensor may be configured for implanting into a blood vessel, e.g. a pulmonary artery. However, it may also be possible that the pressure sensor is configured for implanting into a heart.

As a further example, the implantable device may comprise a cardiac rhythm monitor. Particularly patients that require cardiac rhythm monitoring may also have an increased need for intra-body pressure measurements, such as blood pressure measurements. In other words, patients into which an implantable pressure sensor is implanted may likely also need a cardiac rhythm monitoring device. Since the latter may be implanted subcutaneously, it may be beneficial to additionally adapt it to work as a relay device, providing low loss communication to the outside world (e.g. with a mobile communication apparatus), even though a relatively lossy protocol (when used inside the body) is used.

In an example, the method may further comprise acquiring, by the mobile communication apparatus, second pressure data by a pressure sensor of the mobile communication apparatus. Moreover, it may include correcting the first pressure data at least in part based on the second pressure data and/or forwarding the first and second pressure data for correcting to a remote communication system. In short, the first pressure data may be corrected for variations due to, e.g. ambient pressure variations that are reflected in second pressure data by a corresponding ambient pressure sensor of the mobile communication apparatus. The correction may be carried out, e.g. directly by the mobile communication apparatus. Additionally or alternatively, the correction may be carried out by a remote communication system based on the first and second data forwarded by the mobile communication apparatus.

A sixth aspect relates to an implantable device for assisting in determining an intra-body pressure. The implantable device may comprise means for wirelessly receiving, from an implantable pressure sensor, first pressure data relating to the intra-body pressure acquired by the implantable pressure sensor. It may further comprise means for wirelessly relaying the first pressure data to a mobile communication apparatus for correction of the first pressure data.

The sixth aspect may also be combined with the mobile communication apparatus to provide a corresponding system. The mobile communication apparatus may comprise a pressure sensor for acquiring second pressure data. The mobile communication apparatus may further comprise means for correcting the first pressure data at least in part based on the second pressure data and/or for forwarding the first and second pressure data for correcting to a remote communication system.

It is noted that the methods as described herein may include all aspects described herein, even if not expressly described as method steps but rather with reference to an apparatus or device (or system). Moreover, the apparatuses and devices (and corresponding systems) as outlined herein may include means for implementing all aspects as outlined herein, even if these may rather be described in the context of method steps.

It is noted that the aspects described herein may generally be implemented with a system comprising an implantable pressure sensor and an implantable device, as described herein, and only a general purpose smartphone (embodying the mobile communication apparatus) that is provided with the functionality as described herein via a corresponding app.

It may also be possible to implement the aspects without the implantable device serving as a relay. In that scenario, the implantable pressure sensor would communicate directly with the mobile communication apparatus, and the implantable pressure sensor would be provided with a corresponding communication unit (e.g. Bluetooth Low Energy, and/or NFC). However, this would typically strongly reduce signal quality.

Whether described as method steps, computer program and/or means, the functions described herein may be implemented in hardware, software, firmware, and/or combinations thereof. If implemented in software/firmware, the functions may be stored on or transmitted as one or more instructions or code on a computer-readable medium. Computer-readable media include both computer storage media and communication media including any medium that facilitates transfer of a computer program from one place to another. A storage medium may be any media that can be accessed by a general purpose or special purpose computer and/or processor. By way of example, and not limitation, such computer-readable storage media can comprise RAM, ROM, EEPROM, FPGA, CD/DVD or other optical disk storage, magnetic disk storage or other magnetic storage devices, or any other medium that can be used to carry or store desired program code in the form of instructions or data structures and that can be accessed by a general-purpose or special-purpose computer, or a general-purpose or special-purpose processor.

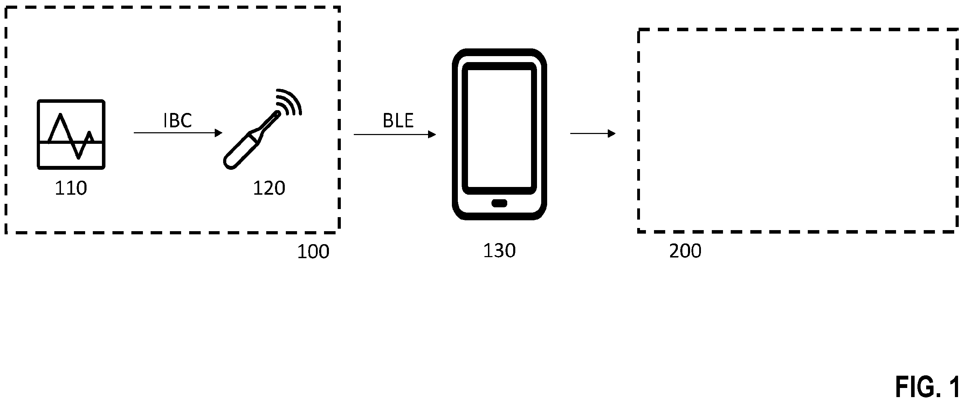

- Fig. 1:: Example of an embodiment of a system according to the present disclosure.

Fig. 1 shows a schematic of an exemplary system according to the present invention.

The system of Fig. 1 comprises a first subsystem 100 that is implantable (or implanted) into a body of a patient and a mobile communication apparatus 130. It may further comprise a remote communication system 200.

Subsystem 100 comprises an implantable pressure sensor 110 that may be implemented as an intravascular pressure sensor. Moreover, subsystem 100 comprises an implantable device 120 that may be considered as a relay device. However, implantable device 120 may have additional functionality, such as providing cardiac rhythm management.

Pressure sensor 110 may comprise a sensor unit that transforms pressure into an electrical signal, for example. It may further comprise an analog to digital converter such that first pressure data may be provided as digital data. Optionally, pressure sensor 110 may provide the first pressure data with timing information. To this end, pressure sensor 110 may comprise an oscillator or a clock, etc. Implantable pressure sensor 110 may further comprise a communication unit to transmit the first pressure data to implantable device 120 (e.g. including an antenna, an RF unit, a modulation/demodulation unit, and/or a signal processing unit). The communication unit may be based on any type of intra-body communication (IBC), preferably a low power communication, for example based on the Medical Implant Communication Service (MICS) or any other system based on electromagnetic signals or also based on ultrasound and/or any other non-electromagnetic signals.

Implantable device 120 may comprise a corresponding first communication unit to receive the first pressure data from pressure sensor 110 (e.g. including an antenna, an RF unit, a modulation/demodulation unit, and/or a signal processing unit). Implantable device may comprise a second communication unit such that the first pressure data may be forwarded to wireless communication apparatus 130 (e.g. including an antenna, an RF unit, a modulation/demodulation unit, and/or a signal processing unit). For example, the second communication unit may operate with Bluetooth Low Energy (BLE). However, additionally or alternatively, also other systems may be supported by the communication unit of implantable device 120 and/or further communication units of implantable device 120, such as Near Field Communication (NFC) and/or any other wireless communication technology. For example, implantable device 120 may comprise hardware and/or a processor with corresponding software/firmware to implement the conversion of the first pressure data as received from the pressure sensor 110 to the format required for transmission to the mobile communication apparatus 130.

Additionally or alternatively to pressure sensor 110, implantable device 120 may provide the first pressure data with timing information. For example, implantable device 120 may comprise an oscillator or a clock, etc.

Both implantable device 120 and implantable pressure sensor 110 may comprise a battery that may optionally be re-chargeable, e.g. via corresponding electromagnetic signals.

The first pressure data may be acquired by the pressure sensor 110 continuously. However, it may be preferable that pressure sensor 110 acquires pressure data only in selected intervals. For example, first pressure data may be acquired for a period of time of 0.5 s to 200 s, 1 s to 100 s, 5 s to 50 s, and/or about 10 s to 30 s. For example, first pressure data may be acquired in such a period of time once a day, once per hour, etc.

The first pressure data may then be transmitted to implantable device 120 directly upon the first pressure data having been acquired, for example. Additionally or alternatively, implantable pressure sensor 110 may store the first pressure data and only forward it to implantable device 120 upon special request, or bundled, e.g. when first pressure data acquired in a certain number of periods of time is available, and/or once a day, once a week, etc.

Implantable device 120 may buffer the received first pressure data but otherwise forward it to mobile communication apparatus 130 immediately. Additionally or alternatively, implantable device 120 may store the received first pressure data and only forward it to mobile communication apparatus 130 upon special request, or bundled, e.g. when first pressure data acquired in a certain number of periods of time is available, and/or once a day, once a week, etc.

Mobile communication apparatus 130 may be implemented as a smartphone, for example. It may comprise a battery, an input/output interface (e.g. a touch-screen and/or one or more physical buttons, etc.), and a processor. It may be owned and/or carried by the patient which has implantable device 120 and pressure sensor 110 into his/her body.

The first pressure data may be influenced by the ambient pressure around the patient, but potential fluctuations due to ambient pressure changes are typically not of interest but rather considered as undesirable. Hence, the first pressure data may be corrected by mobile communication apparatus and/or remote communication system 200, as outlined in the following:
Mobile communication apparatus 130 may comprise a pressure sensor, e.g. for sensing ambient pressure around the patient. The pressure sensor may be enclosed in the housing of mobile communication apparatus 130, for example. Hence, apart from receiving first pressure data from implantable device 120 (e.g. via a corresponding communication unit), mobile communication apparatus 130 may receive second pressure data from its own pressure sensor.
Mobile communication apparatus 130 may continuously receive second pressure data such that the ambient pressure is continuously recorded. This may allow correcting corresponding first pressure data (having a matching timing information) even if the first pressure data is only received by the mobile communication apparatus 130 at a later time (e.g. as described above).

The second pressure data may be provided by the pressure sensor of the mobile communication apparatus 130 with timing information. Additionally or alternatively, the second pressure data may be received at a processing unit (e.g. the processor, a microcontroller, an ASIC, etc.) of communication apparatus 130 that may provide the second pressure data with corresponding timing information.

In both cases, the processing unit may correct the first pressure data based on the second pressure data to provide corrected first pressure data. For example, it may carry out a subtraction including first and second pressure data. For example, a pressure value of the second pressure data may simply be subtracted from a corresponding pressure value (e.g. corresponding to the same or similar timing information as the pressure value of the second pressure data) of the first pressure data. Hence, pressure variations due to pressure variations of the ambient pressure would be removed. Mobile communication apparatus 130 may comprise a storage medium to store the first pressure data, the second pressure data, and/or the corrected first pressure data. Additionally or alternatively, it may send the first pressure data, the second pressure data, and/or the corrected first pressure data to remote communication system 200, possibly as a joint dataset or as separate datasets.

Additionally or alternatively, mobile communication apparatus 130 may simply forward the first pressure data and the second pressure data (both with timing information, e.g. as outlined above) to remote communication system 200, possibly as a joint dataset or as two separate datasets. The provision of corrected first pressure data may then be implemented by remote communication system 200.

Regardless of whether the provision of corrected first pressure data is carried out by mobile communication apparatus 130 and/or remote communication system 200, the latter may for example be implemented as a cloud or server based system which stores and monitors corrected first pressure data. Communication between mobile communication apparatus 130 and remote communication system 200 may be implemented in a wired and/or wireless manner, e.g. involving the internet, e.g. over a 3G, 4G, 5G or WiFi interface of mobile communication apparatus 130. For example, remote communication system 200 may handle corrected first pressure data associated with a plurality of patients that may transmit data to remote communication system 200 via their respective mobile communication apparatuses 130.

Remote communication system 200 may monitor the data received from mobile communication apparatuses 130 for consistency, e.g. whether the data is plausible. Additionally or alternatively, it may monitor the corrected first pressure data such as to monitor the patient's health state. Based thereon, various actions may be triggered, e.g. the patient may be invited for a check-up, if suspicious pressure values are noted. Also, it is possible to take emergency measures, e.g. send an alarm to mobile communication apparatus 130 and/or directly send medical personnel to the patient associated with mobile communication apparatus 130 in case a serious condition is detected that requires immediate attention. The monitoring may be done automatically and/or based on decisions of medical staff that has access to the remote communication system 200.

Although described with respect to remote communication system 200, at least the automatic monitoring functions may additionally or alternatively be provided by mobile communication apparatus 130 itself. In some examples, the described overall system even may not comprise the optional remote communication system 200.

The functionality of mobile communication apparatus 130 described herein may be implemented by means of a corresponding app that may be downloaded to mobile communication apparatus 130 that may otherwise be a general purpose device, e.g. a (general purpose) smartphone.

## Claims

1. A method for determining an intra-body pressure, comprising:
acquiring first pressure data relating to the intra-body pressure by an implantable pressure sensor (110);
wirelessly transmitting the first pressure data to an implantable device (120);
wirelessly relaying, by the implantable device, the first pressure data to a mobile communication apparatus (130) for correction of the first pressure data.

2. The method of claim 1, further comprising:
acquiring, by the mobile communication apparatus (130), second pressure data by a pressure sensor of the mobile communication apparatus;
correcting the first pressure data at least in part based on the second pressure data and/or forwarding the first and second pressure data for correcting to a remote communication system (200).

3. A system (100) for determining an intra-body pressure, comprising:
an implantable pressure sensor (110) for acquiring first pressure data relating to the intra-body pressure;
an implantable device (120) comprising means for wirelessly receiving the first pressure data from the implantable pressure sensor and for wirelessly relaying the first pressure data to a mobile communication apparatus (130) for correction of the first pressure data.

4. The system (100) of claim 3, further comprising the mobile communication apparatus (130), wherein the mobile communication apparatus comprises a pressure sensor for acquiring second pressure data;
wherein the mobile communication apparatus comprises means for correcting the first pressure data at least in part based on the second pressure data and/or for forwarding the first and second pressure data for correcting to a remote communication system (200).

5. A method for determining an intra-body pressure, carried out by a mobile communication apparatus (130), comprising:
wirelessly receiving, from a relay device (120), first pressure data relating to the intra-body pressure acquired by an implantable pressure sensor (110);
receiving second pressure data acquired by a pressure sensor of the mobile communication apparatus;
correcting the first pressure data at least in part based on the second pressure data and/or forwarding the first and second pressure data for correcting to a remote communication system (200).

6. The method of claim 5, wherein the wirelessly receiving comprises wirelessly receiving via at least one of: Bluetooth and Near Field Communication.

7. The method of claim 5 or 6, wherein the mobile communication apparatus is a handheld apparatus, in particular a smartphone.

8. The method of any of claims 5-7, wherein the relay device is an implantable device.

9. A computer program comprising instructions that, when carried out by a processor of a mobile communication apparatus (130), causes the mobile communication apparatus to implement the steps according to the method of any of claims 5-8.

10. A method for assisting in determining an intra-body pressure, carried out by an implantable device (120), comprising:
wirelessly receiving, from an implantable pressure sensor (110), first pressure data relating to the intra-body pressure acquired by the implantable pressure sensor;
wirelessly relaying the first pressure data to a mobile communication apparatus (130) for correction of the first pressure data.

11. The method of any of claims 1, 2 or 10, wherein the wirelessly relaying comprises wirelessly relaying via at least one of: Bluetooth and Near Field Communication.

12. The method of any of claims 1, 2 or 10, 11 wherein the mobile communication apparatus (130) is embodied as a handheld apparatus, in particular a smartphone.

13. The method of any of claims 1, 2 or 10-12, wherein the implantable pressure sensor (110) comprises an intravascular blood pressure sensor.

14. The method of any of claims 1, 2 or 10-13, wherein the implantable device (120) comprises a cardiac rhythm monitor.

15. An implantable device (120) for assisting in determining an intra-body pressure, comprising:
means for wirelessly receiving, from an implantable pressure sensor (110), first pressure data relating to the intra-body pressure acquired by the implantable pressure sensor;
means for wirelessly relaying the first pressure data to a mobile communication apparatus (130) for correction of the first pressure data.
